(19) Europäisches Patentamt
European Patent Office
Office européen des brevets

(11) Veröffentlichungsnummer : **0 090 341**
**B1**

(12) # EUROPÄISCHE PATENTSCHRIFT

(45) Veröffentlichungstag der Patentschrift : **29.07.87**

(51) Int. Cl.[4] : **A 61 K 37/02,** C 07 D209/54, C 07 K 5/06

(21) Anmeldenummer : **83102868.3**

(22) Anmeldetag : **23.03.83**

(54) **Spiro(4.(3+n))-2-aza-alkan-3-carbonsäure-Derivate, Verfahren zu ihrer Herstellung, diese enthaltende Mittel und ihre Verwendung.**

(30) Priorität : **27.03.82 DE 3211397**

(43) Veröffentlichungstag der Anmeldung : **05.10.83 Patentblatt 83/40**

(45) Bekanntmachung des Hinweises auf die Patenterteilung : **29.07.87 Patentblatt 87/31**

(84) Benannte Vertragsstaaten : **AT BE CH DE FR GB IT LI LU NL SE**

(56) Entgegenhaltungen :
**EP-A- 0 012 401**
**EP-A- 0 037 231**
**EP-A- 0 050 800**
**EP-A- 0 094 633**

(73) Patentinhaber : **HOECHST AKTIENGESELLSCHAFT**
**Postfach 80 03 20**
**D-6230 Frankfurt am Main 80 (DE)**

(72) Erfinder : **Teetz, Volker, Dr.**
**An der Tann 20**
**D-6238 Hofheim am Taunus (DE)**
Erfinder : **Urbach, Hansjörg, Dr.**
**Le Lavandoustrasse 41**
**D-6242 Kronberg Taunus (DE)**
Erfinder : **Becker, Reinhard, Dr.**
**Adelheidstrasse 101**
**D-6200 Wiesbaden (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die am 5. Mai 1982 veröffentlichte Europäische Patentanmeldung Veröffentlichungsnummer EP-A1-50 800 betrifft Carboxyalkyl-Dipeptide, Verfahren und Zwischenprodukte zu deren Herstellung sowie deren Verwendung als Inhibitoren des Angiotensin Converting Enzymes (ACE).

Es wurden neue spirocyclische 2-Azaalkancarbonsäure-Derivate gefunden, die eine langandauernde, intensiv blutdrucksenkende Wirkung besitzen.

Die Erfindung betrifft daher Verbindungen der Formel I

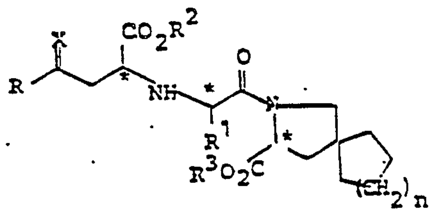

(I)

in welcher n 1, 2, 3 oder 4 ; R Phenyl ; $R^1$ einen Alkylrest einer natürlich vorkommenden Aminosäure HOOC—CH($NH_2$)—$R^1$ ; $R^2$ Wasserstoff, Alkyl mit 1 bis 6 C-Atomen oder gegebenenfalls nitrosubstituiertes Aralkyl mit 7 bis 9 C-Atomen ; $R^3$ Wasserstoff, Alkyl mit 1 bis 10 C-Atomen, Cycloalkyl mit 3 bis 10 C-Atomen oder gegebenenfalls nitrosubstituiertes Aralkyl mit 7 bis 9 C-Atomen und X zwei Wasserstoffatome oder 1 Sauerstoffatom bedeuten, sowie deren physiologisch verträgliche Salze mit Säuren und, falls $R^2$ und/oder $R^3$ Wasserstoff bedeuten, mit Basen, wobei 2-[N-(1-Carboethoxy-3-phenylpropyl)-(S)-alanyl]-2-azaspiro [4.4] nonan-3(S)-carbonsäure und 2-[N-(1-Carboethoxy-3-phenylpropyl)-(S)-alanyl]-2-azaspiro [4.5] decan-3(S)-carbonsäure sowie deren physiologisch verträglichen Salze ausgenommen sind.

In Formel I können das C-Atom in Position 3 der Spirocyclus und die mit einem Stern (*) markierten C-Atome der Kette sowohl R- als auch S-Konfigurationen aufweisen. Bevorzugt sind jedoch Verbindungen, deren C-Atom in Position 3 des Spirocyclus und deren mit einem Stern markierten C-Atome der Kette jeweils die S-Konfiguration aufweisen.

Besonders bevorzugt sind solche Verbindungen der Formel I, in welcher, R und X vorstehende Bedeutungen haben und $R^1$ Methyl, $R^2$ Wasserstoff, Alkyl mit 1 bis 4 C-Atomen, Benzyl oder Nitrobenzyl und $R^3$ Wasserstoff bedeuten.

Die Erfindung betrifft weiterhin ein Verfahren zur Herstellung von Verbindungen der Formel I, das dadurch gekennzeichnet ist, daß man Verbindungen der Formel II

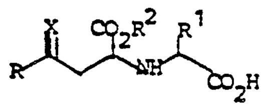

(II)

in welcher R, $R^1$, $R^2$ und X die vorstehend angegebenen Bedeutungen haben mit Verbindungen der Formel III,

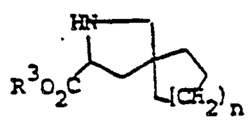

(III)

in welcher $R^3$ die vorstehend angegebene Bedeutung mit Ausnahme der von Wasserstoff hat und n für eine ganze Zahl von 1 bis 4 steht, kondensiert und anschließend gegebenenfalls die Reste $R^2$ und/oder $R^3$ hydrogenolytisch, sauer oder basisch abspaltet und die erhaltenen Verbindungen der Formel I gegebenenfalls in ihre physiologisch verträglichen Salze überführt.

Die Kondensation der Verbindungen der Formel II mit den Estern der Formel III erfolgt vorzugsweise nach bekannten Verfahren der Peptidchemie. Besonders bevorzugt sind solche Verfahren, die ausreichend Schutz vor Racemisierung bieten, wie z. .B. die DCC/HOBt-Methode oder die in der DE-OS 29 01 843 beschriebene Alkanphosphonsäureanhydrid-Methode. Als Reste $R^3$ der oben genannten Ester kommen beispielsweise hydrogenolytisch abspaltbare Gruppen (s. z. B. Org. Reactions I (1953) 272), vorzugsweise Benzyl oder Nitrobenzyl, oder sauer bzw. basisch abspaltbare Gruppen (s. z. B. Houben-Weyl, Bd. 8 (1952)) vorzugsweise tert.-Butyl, in Frage.

Verbindungen der Formel I, in denen wenigstens einer der Reste $R^2$ und $R^3$ Wasserstoff ist, können nach bekannten Methoden in die Ester der Formel I überführt werden, in denen $R^2$ und $R^3$ die oben genannte Bedeutung mit Ausnahme der von Wasserstoff haben.

Verfahren zur Herstellung von Verbindungen der Formel II wurden bereits vorgeschlagen.

Die Erfindung betrifft ferner ein Verfahren zur Herstellung von Verbindungen der Formel III, in welcher n = 1, 2, 3 und 4 und $R^3$ Wasserstoff, Alkyl oder Cycloalkyl mit 1 bis 10 C-Atomen oder ggf.

nitrosubstituiertes Aralkyl mit 7 bis 9 C-Atomen bedeuten, das dadurch gekennzeichnet ist, daß man Verbindungen der Formel IV

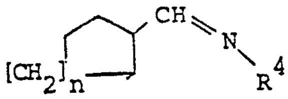

(IV)

in welcher n die vorstehende Bedeutung hat und $R^4$ für Alkyl mit 1 bis 6 C-Atomen steht, mit einem metallorganischen Reagenz in einem inerten Lösungsmittel metalliert und anschließend mit Verbindungen der Formel V,

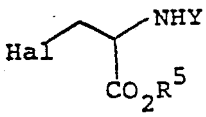

(V)

in welcher Hal Brom oder Chlor, $R^5$ Alkyl mit 1 bis 6 C-Atomen oder Aralkyl mit 7 bis 9 C-Atomen und Y einen sauer abspaltbaren aliphatischen oder aromatischen Acylrest bedeuten, oder mit Verbindungen der Formel VI,

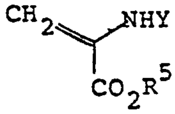

(VI)

in welcher $R^5$ und Y vorstehende Bedeutung haben, zu Verbindungen der Formel VII,

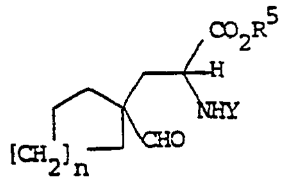

(VII)

in welcher n, $R^5$ und Y vorstehende Bedeutung haben, umsetzt, diese durch Säurebehandlung zu Verbindungen der Formel VIII,

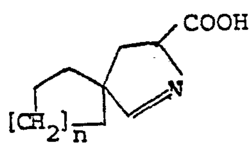

(VIII)

in welcher n die vorstehende Bedeutung hat, cyclisiert, diese zu Verbindungen der Formel III, in welcher n vorstehende Bedeutung hat und $R^3$ für Wasserstoff steht, reduziert und diese anschließend zu Verbindungen der Formel III, in welcher n vorstehende Bedeutung und $R^3$ die vorstehend angegebene Bedeutung mit Ausnahme der von Wasserstoff haben, verestert.

Die Schiff'schen Basen (Formel IV) aus einem Alkylamin $R^4$-$NH_2$ wie beispielsweise Butylamin und einem entsprechenden Cycloalkanaldehyd mit 5 bis 8 Ring-C-Atomen werden in einem inerten Lösungsmittel, wie z. B. Diethylether, THF oder Dimethoxyäthan mit einer metallorganischen Verbindung in ihr Carbanion überführt.

Zu den N-acylierten Halogenserinestern der Formel V bzw. entsprechenden Acrylestern der Formel VI ist Y vorzugsweise ein sauer abspaltbarer Acylrest, z. B. Acetyl oder tert. Butyloxycarbonyl und $R^5$ bedeutet vorzugsweise Methyl, jedoch auch anderes Alkyl oder Aralkyl.

Die Verbindungen der Formel VII fallen als Racemate an. Sie werden in Säuren, vorzugsweise wäßrigen Mineralsäuren, wie beispielsweise etwa 2 n Salzsäure unter gleichzeitiger Entacyclierung und Esterspaltung zu Verbindungen der Formel VII cyclisiert.

Die Reduktion der Verbindungen der Formel VII kann sowohl durch katalytische Hydrierung (beispielsweise an Pt/C unter Normalbedingungen) oder mit komplexen Borhydriden oder Boran-Aminkomplexen vorzugsweise in niederen Alkoholen erfolgen.

Die Aminosäuren der Formel III werden nach an sich bekannten Methoden (vgl. z. B. Houben-Weyl, Bd. 8 [1952] S. 359-680) in die entsprechenden Ester überführt.

Sie fallen als Salze von Mineralsäuren oder anderen starken Säuren, z. B. Toluolsulfonate an und können als solche oder in Form der freien Basen in die nachfolgende Peptidkupplung eingesetzt werden. Ein spezieller Schutz des Imino-Stickstoffs in Verbindung der Formel II ist hierbei nicht notwendig.

Man erhält Bisester der Formel I, aus denen sich je nach gewählter Esterkombination durch saure, basische und/oder hydrogenolytische Methoden (im Falle X = Sauerstoff z. B. an $Pd/BaSO_4$ in DMF) die Monoester oder Dicarbonsäuren gemäß Formel I herstellen lassen. Eine Trennung der entstehenden Diastereomeren ist sowohl bei den Bisestern der Formel I als auch bei den Monoestern oder Dicarbonsäuren der Formel I durch chromatographische Verfahren, z. B. Säulenchromatographie an Kieselgel, möglich. In Einzelfällen gelingt eine fraktionierte Kristallisation der Verbindungen, meist in Form ihrer mineralsauren Salze (z. B. ihrer Hydrochloride) zu diastereomeren-reinen Produkten.

Die Erfindung betrifft weiterhin Verbindungen der Formel IX,

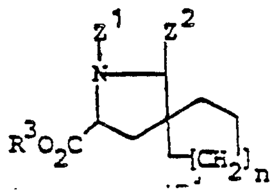

(IX)

in welcher n und $R^3$ die obengenannte Bedeutung haben und $Z^1$, $Z^2$ jeweils für Wasserstoff oder gemeinsam für eine chemische Bindung stehen, wobei Verbindungen der Formel IX ausgenommen sind, worin n = 1 ist, $R^3$ Wasserstoff oder Ethyl bedeutet und $Z^1$, $Z^2$ jeweils für Wasserstoff steht.

Die neuen Verbindungen der allgemeinen Formel I besitzen eine langandauernde, intensive blutdrucksenkende Wirkung. Sie werden nach peroraler Gabe gut resorbiert und können zur Bekämpfung des Bluthochdrucks verschiedener Genese eingesetzt und für sich oder in Kombination mit anderen blutdrucksenkenden, gefäßerweiternden oder diuretisch wirksamen Verbindungen angewandt werden. Die Anwendung kann intravenös, subcutan oder peroral erfolgen, die perorale Gabe wird bevorzugt. Die Dosierung bei peroraler Gabe liegt bei 0,1-100 mg je Einzeldosis, vorzugsweise 1-50 mg, insbesondere 1-30 mg für einen normalgewichtigen Erwachsenen. Dies entspricht einer Dosis von 1-1 300 µg/kg/Tag, vorzugsweise 13-700 µg/kg/Tag, insbesondere 13-400 µg/kg/Tag.

Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden. Bei intravenöser oder subcutaner Verabreichung sollte die Einzeldosis zwischen 0,01 und 20 mg liegen.

Im Tierversuch wurden z. B. folgende Wirksamkeiten erhalten : Es wurde die Unterdrückung des durch 310 ng Angiotensin I ausgelösten Pressorresponses an der Ratte gemessen.

a) i. v. Applikation (30 Min. nach Applikation)

| Verbindung I | | | | | | | |
|---|---|---|---|---|---|---|---|
| R | $R^1$ | $R^2$ | $R^3$ | n | X | Dosis | Hemmung |
| $C_6H_5$ | $CH_3$ | $C_2H_5$ | H | 1 | $=H_2$ | 100 µg/kg | 95 % |
| $C_6H_5$ | $CH_3$ | $C_2H_5$ | H | 2 | $=H_2$ | 100 µg/kg | 95 % |
| $C_6H_5$ | $CH_3$ | $C_2H_5$ | H | 2 | = O | 100 µg/kg | 95 % |
| $C_6H_5$ | $CH_3$ | H | | 3 | $=H_2$ | 100 µg/kg | 95 % |

b) i. d. Applikation (30 Min. nach Applikation)

| R | $R^1$ | $R^2$ | $R^3$ | n | X | Dosis | Hemmung |
|---|---|---|---|---|---|---|---|
| $C_6H_5$ | $CH_3$ | $C_2H_5$ | H | 1 | $= H_2$ | 1 mg/kg | 85-95 % |
| $C_6H_5$ | $CH_3$ | $C_2H_5$ | H | 2 | $= H_2$ | 0,1 mg | 60-70 % |

Die Verbindungen der Formel I mit $R^3$ = Wasserstoff liegen als innere Salze vor. Falls beide Carboxylgruppen frei sind, können zusätzlich Alkali- und Calcium-, Magnesium- und Zinksalze und solche mit physiologisch unbedenklichen Aminen gebildet werden. Ferner kann die freie Aminogruppe mit einer Mineralsäure wie HCl, HBr, $H_3PO_4$, $H_2SO_4$ oder organischen Säure wie Citronensäure, Weinsäure, Maleinsäure, Fumarsäure zu einem Salz umgesetzt werden.

Die nachfolgenden Beispiele sollen die erfindungsgemäßen Verfahrensweisen erläutern, ohne die Erfindung auf die hier stellvertretend genannten Substanzen zu beschränken. Von allen einschließlich der in den Beispielen nicht erwähnten Verbindungen wurden zufriedenstellende Analysen (CHN-Analysen, NMR-Spektren) erhalten.

Beispiel 1

Spiro [4.5]-2-aza-decan-3-carbonsäure

16.7 g Butyliminomethylcyclohexan in 300 ml wasserfreiem Dimethoxyethan werden mit 6,4 g Butyllithium (in Form einer Hexan-Lösung) bei — 76 °C unter Schutzgas (Argon) versetzt. 15 Min. nach beendeter Zugabe werden unter guter Kühlung 18 g N-Acetyl-chlorserinmethylester in 100 ml DME eingetropft. Man rührt noch 1 Stunde bei tiefer Temperatur, läßt erwärmen und engt im Vakuum weitgehend ein. Das Rohgemisch wird mit 200 ml 2n HCl angesäuert und 45 Min. zum Rückfluß erhitzt. Man engt im Vakuum ein, nimmt den Rückstand in Eisessig auf, versetzt mit 1 g Pt/C (10 % Pt) und hydriert bei Normaldruck. Man filtriert, engt ein und chromatographiert über eine Kieselgelsäule im System Chloroform/Methanol/Eisessig/Wasser 25/15/2/2. Aus dem entsprechenden Eluat kristallisiert nach Einengen die Aminosäure. Schmp. 205 °C (Zers.) Rf : 0,51 (Kieselgelplatten, System wie für Säulenchromatographie)

Beispiel 2

(S,S,S)-N-(1-Carbethoxy-3-phenyl-propyl)-alanyl-spiro [4.4]-2-aza-nonan-3-carbonsäure

14,8 g (D,L)-Spiro [4,4]-2-aza-nonan-3-carbonsäurebenzylester-hydrochlorid, 6,7 g 1-Hydroxybenzotriazol und 13,8 g (S,S)-N-(1-Carbethoxy-3-phenyl-propyl)-alanin werden in 200 ml DMF gelöst und mit 10,2 g Dicyclohexylcarbodiimid über Nacht zur Reaktion gebracht. Zusatz von tert. Basen, z. B. 6,4 ml N-Ethylmorpholin erhöht die Ausbeute nur unwesentlich. Man filtriert vom ausgefallenen DC-Harnstoff ab, engt das Filtrat im Vakuum ein, nimmt in Essigester auf, schüttelt mit wäßriger Natrium-bikarkonatlösung aus, trocknet die organische Phase über festem Natriumsulfat und engt erneut ein. Der Rückstand wird über 1 kg Kieselgel (60 Å-Porenweite) im System Essigester (Petrolether 2 : 1 säulenchromatographisch getrennt. Es wird zunächst das (S,S,S)- nachfolgend das (S,S,R)-Isomere eluiert. Dünnschichtchromatographie an Kieselgelplatten im obigen Laufmittelsystem : Rf = 0,7 bzw. 0,6. Nach Einengen der entsprechenden Eluate werden die Verbindungen alz zähe Öle erhalten. Die NMR-Spektren (in $CDCl_3$) bestätigen die Struktur.

Beispiel 3

(S,S,S)- bzw. (S,S,R)-N-(1-Carbethoxy-3-phenyl-propyl)-alanyl-spiro [4.4]-2-aza-nonan-3-carbonsäure

Die in Beispiel 2 erhaltenen diastereomeren Benzylester werden in 200 ml Methanol aufgenommen und mit 1 g Pd/C (10 % Pd) hydrogenolytisch entbenzyliert. Nach beendeter Wasserstoffaufnahme wird filtriert und im Vakuum eingeengt. Unter Zusatz von Pentan kann im Vakuum ein fester, hygroskopischer Schaum der zwitterionischen Dipeptid-derivate erhalten werden.

S,S,S-Verbindung : $[\alpha]_D^{20}$ = — 78,9 (c = 1,5, Methanol)

S,S,R-Verbindung : $[\alpha]_D^{20}$ = + 28,4 (c = 1, Methanol)

Die entsprechenden Spiro [4,5]-, [4,6] und [4,7]-2-azaalkan-3-carbonsäurederivate werden auf analoge Weise erhalten. Die Drehwerte für die (S,S,S) bzw. (S,S,R)-N-(1-Carbethoxy-3-phenylpropyl)-alanyl-spiro [4,5]-2-aza-decan-3-carbonsäure betragen.

z. B. (S,S,S)-Verbindung : $[\alpha]_D^{21}$ = — 38,3 (c = 1, Methanol)

(S,S,R)-Verbindung : $[\alpha]_D^{21}$ = + 18,5 (c = 1, Methanol)

Beispiel 4

N-(1-Carbethoxy-3-oxo-3-phenyl-propyl)-alanyl-spiro-[4,5]-2-aza-decan-3-carbonsäure

Die Verbindung kann auf verschiedenen Wegen erhalten werden :

a) In Analogie zu Beispiel 2 und 3. Die Spaltung des Benzylesters erfolgt zweckmäßigerweise mit Pd/$BaSO_4$ in DMF um eine unerwünschte Reduktion der zum Phenylring α-ständigen Ketogruppe zu vermeiden. Die Hydrierung wird nach Beendigung der rasch verlaufenden Benzylesterspaltung abgebrochen.

b) Unter Verwendung von Spiro [4.5]-2-aza-decan-3-carbonsäure-tert.-butylester zur Peptidkupplung und nachfolgende saure Esterspaltung mit HCl/Dioxan oder wasserfreier Trifluoressigsäure entsprechend bekannten Methoden der Peptidchemie.

Beispiel 5

N-(1-Carboxy-3-phenyl-propyl)-alanyl-spiro [4.4]-2-aza-nonan-3-carbonsäure

1 g des Ethylesters aus Beispiel 3 wird in 25 ml Dioxan aufgenommen und mit einer äquivalenten Menge 2n Natronlauge verseift. Man stellt mit wenig Salzsäure auf pH 4, engt zur Trockene ein, versetzt mit wenig gesättigter NaCl-Lösung und extrahiert die Titelverbindung mit n-Butanol. Ausbeute : 700 mg. Rf : 0,31 (Kieselgel, System : $CHCl_3$/Methanol/Eisessig 50 : 10 : 2).

Beispiel 6

Allgemeine Vorschrift zur Synthese der Spiro-Aminosäurebenzylester-hydrochloride :

Bei — 5 °C werden 50 g Thionylchlorid in 300 ml Benzylalkohol eingetropft. Man rührt 30 Min. nach und fügt dann 0,2 Mol Aminosäure-hydrochlorid zu. Nach 6-10 Stunden bei Raumtemperatur ist die Reaktion beendet. Die Reaktionslösung wird im Vakuum eingeengt und der Rückstand mit Diisopropyläther gefällt. Auf diese Weise ist zum Beispiel Spiro [4.5]-2-aza-decan-3-carbonsäure-benzylester-hydrochlorid erhältlich : Schmp. 145 °C, Rf = 0,71 (Kieselgel, System : $CHCl_3/CH_3OH/CH_3COOH$ 50/10/2).

**Patentansprüche** (für die Vertragsstaaten : BE, DE, GB, FR, IT, LU, NL, SE, CH)

1. Verbindungen der Formel I

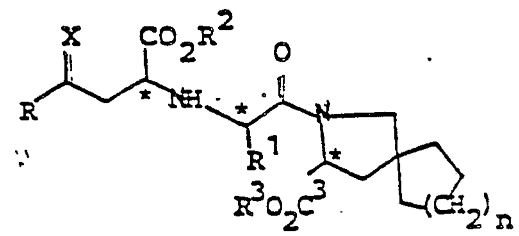

in welcher

n 1, 2, 3 oder 4,

$R_1$ Phenyl,

$R^1$ einen Alkylrest einer natürlich vorkommenden Aminosäure HOOC—CH($NH_2$)—$R^1$,

$R^2$ Wasserstoff, Alkyl mit 1 bis 6 C-Atomen oder gegebenenfalls nitrosubstituiertes Aralkyl mit 7 bis 9 C-Atomen,

$R^3$ Wasserstoff, Alkyl mit 1 bis 10 C-Atomen, Cycloalkyl mit 3 bis 10 C-Atomen der gegebenenfalls nitrosubstituiertes Aralkyl mit 7 bis 9 C-Atomen und

X 2 Wassserstoffatome oder 1 Sauerstoffatom bedeuten, sowie deren physiologisch verträgliche Salze mit Säuren und, falls $R^2$ und/oder $R^3$ Wasserstoff bedeuten, mit Basen,

wobei 2-[N-(1-Carboethoxy-3-phenylpropyl)-(S)-alanyl]-2-azaspiro [4.4]nonan-3(S)-carbonsäure und 2-[N-(1-Carboethoxy-3-phenylpropyl)-(S)-alanyl]-2-azaspiro [4.5] decan-3 (S)-carbonsäure sowie deren physiologisch verträgliche Salze ausgenommen sind.

2. Verbindung der Formel I gemäß Anspruch 1, dadurch gekennzeichnet, daß das C-Atom in 3-Position des Spirocyclus sowie die mit einem Stern markierten C-Atome der Kette jeweils S-Konfiguration aufweisen.

3. Verbindungen der Formel I gemäß den Ansprüchen 1 und 2, dadurch gekennzeichnet, daß

$R^1$ Methyl,

$R^2$ Wasserstoff, Alkyl mit 1 bis 4 C-Atomen, Benzyl oder Nitrobenzyl und

$R^3$ Wasserstoff bedeuten.

4. Verfahren zur Herstellung von Verbindungen der Formel I, dadurch gekennzeichnet, daß man Verbindungen der Formel II

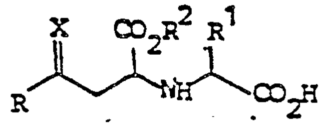

(II)

in welcher $R^1$, $R^2$ und X die in Anspruch 1 angegebenen Bedeutungen haben mit Verbindungen der Formel III,

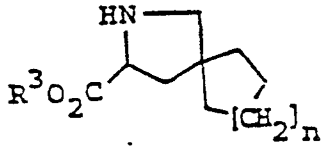

(III)

in welcher $R^3$ die im Anspruch 1 angegebene Bedeutung mit Ausnahme der von Wasserstoff hat und n für eine ganze Zahl von 1 bis 4 steht, kondensiert und anschließend gegebenenfalls die Reste $R^2$ und/oder $R^3$ hydrogenolytisch sauer oder basisch abspaltet und die erhaltenen Verbindungen der Formel I gegebenenfalls in ihre physiologisch verträglichen Salze überführt.

5. Verfahren zur Herstellung von Verbindungen der Formel III, in welcher n und $R^3$ die im Anspruch 1 genannte Bedeutung haben, dadurch gekennzeichnet, daß man Verbindungen der Formel IV

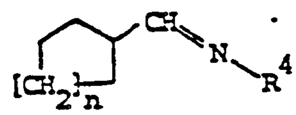

(IV)

in welcher n die vorstehende Bedeutung hat und $R^4$ für Alkyl mit 1 bis 6 C-Atomen steht, mit einem metallorganischen Reagenz in einem inerten Lösungsmittel metalliert und anschließend mit Verbindungen der Formel V

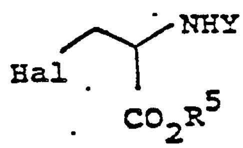

(V)

in welcher Hal Brom oder Chlor, $R^5$ Alkyl mit 1 bis 6 C-Atomen oder Aralkyl mit 7 bis 9 C-Atomen und Y einen sauer abspaltbaren aliphatischen oder aromatischen Acylrest bedeuten, oder mit Verbindungen der Formel VI,

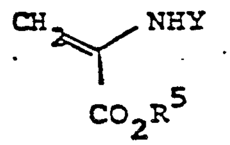

(VI)

in welcher $R^5$ und Y vorstehende Bedeutung haben, zu Verbindungen der Formel VII,

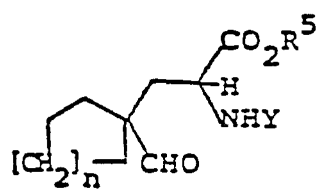

(VII)

in welcher n, $R^5$ und Y vorstehende Bedeutung haben, umsetzt, diese durch Säurebehandlung zu Verbindungen der Formel VIII

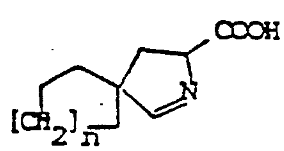

(VIII)

in welcher n die vorstehende Bedeutung hat, cyclisiert, diese zu Verbindungen der Formel III, in welcher n vorstehende Bedeutung hat und $R^3$ für Wasserstoff steht, reduziert und diese anschließend zu Verbindungen der Formel III, in welcher n vorstehende Bedeutung und $R^3$ die im Anspruch 1 angegebene Bedeutung mit Ausnahme der von Wasserstoff haben, verestert.

6. Verbindungen der Formel IX

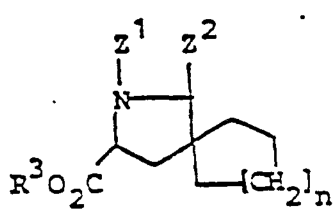

(IX)

in welcher n und $R^3$ die in Anspruch 1 genannte Bedeutung haben und $Z^1$, $Z^2$ jeweils für Wasserstoff oder gemeinsam für eine chemische Bindung stehen, wobei Verbindungen der Formel IX ausgenommen sind, worin n = 1 ist, $R^3$ Wasserstoff oder Ethyl bedeutet und $Z^1$, $Z^2$ jeweils für Wasserstoff steht.

7. Verbindungen der Formel IX gemäß Anspruch 6, dadurch gekennzeichnet, daß $Z^1$ und $Z^2$ Wasserstoff bedeuten.

8. Verbindung gemäß den Ansprüch 1 bis 3 zur Verwendung als Heilmittel.

9. Verbindung gemäß Anspruch 8 zur Verwendung im Kombination mit einem Diuretikum.

10. Arneimittel, enthaltend eine Verbindung gemäß den Ansprüchen 1 bis 3.

11. Arneimittel, enthaltend eine Verbindung gemäß den Ansprüchen 1 bis 3 und ein Diuretikum.

**Patentansprüche** (für den Vertragsstaat AT)

1. Verfahren zur Herstellung von Verbindungen der Formel I

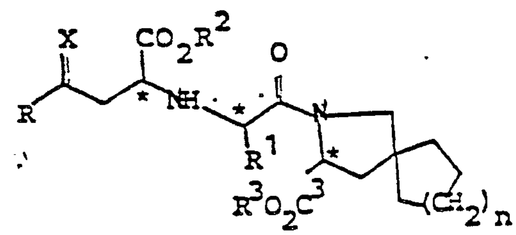

in welcher
n 1, 2, 3 oder 4,
R Phenyl,
$R^1$ einen Alkylrest einer natürlich vorkommenden Aminosäure HOOC—CH($NH_2$)—$R^1$,
$R^2$ Wasserstoff, Alkyl mit 1 bis 6 C-Atomen oder gegebenenfalls nitrosubstituiertes Aralkyl mit 7 bis 9 C-Atomen,
$R^3$ Wasserstoff, Alkyl mit 1 bis 10 C-Atomen, Cycloalkyl mit 3 bis 10 C-Atomen oder gegebenenfalls nitrosubstituiertes Aralkyl mit 7 bis 9 C-Atomen und
X 2 Wasserstoffatome oder 1 Sauerstoffatom bedeuten, sowie deren physiologisch verträglichen Salze mit Säuren und, falls $R^2$ und/oder $R^3$ Wasserstoff bedeuten, mit Basen,
wobei 2-[N-(1-Carboethoxy-3-phenylpropyl)-(S)-alanyl]-2-azaspiro [4.4] nonan-3 (S)-carbonsäure und 2-[N-(1-Carboethoxy-3-phenylpropyl)-(S)-alanyl]-2-azaspiro [4.5] decan-3 (S)-carbonsäure sowie deren physiologisch verträglichen Salze ausgenommen sind, dadurch gekennzeichnet, daß man Verbindungen der Formel II

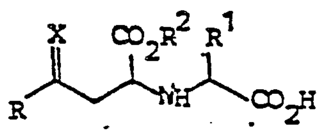

(II)

in welcher R, $R^1$, $R^2$ und X die oben angegebenen Bedeutungen haben mit Verbindungen der Formel III,

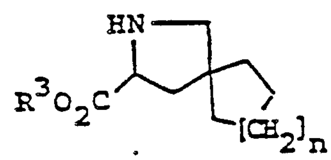

(III)

in welcher $R^3$ die oben angegebene Bedeutung mit Ausnahme der von Wasserstoff hat und n für ein ganze Zahl von 1 bis 4 steht, kondensiert und anschließend gegebenenfalls die Reste $R^2$ und/oder $R^3$ hydrogenolytisch sauer oder basisch abspaltet und die erhaltenen Verbindungen der Formel I gegebenenfalls in ihre physiologisch verträglichen Salze überführt.

2. Verfahren gemäß Anspruch 1, dadurch gekennzeichnet, daß Verbindungen der Formel I hergestellt werden, in denen das C-Atom in 3-Position des Spirocyclus sowie die mit einem Stern markierten C-Atome der Kette jeweils S-Konfiguration aufweisen.

3. Verfahren der Formel I gemäß den Ansprüchen 1 und 2, dadurch gekennzeichnet, daß in Formel I
$R^1$ Methyl,
$R^2$ Wasserstoff, Alkyl mit 1 bis 4 C-Atomen, Benzyl oder Nitrobenzyl und
$R^3$ Wasserstoff bedeuten.

4. Verfahren zur Herstellung von Verbindungen der Formel IX

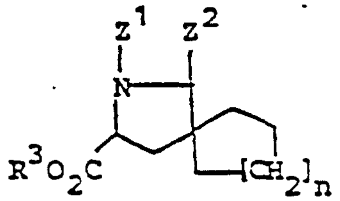

(IX)

in welcher n und $R^3$ die in Anspruch 1 genannte Bedeutung haben und $Z^1$, $Z^2$ jeweils für Wasserstoff oder gemeinsam für eine chemische Bindung stehen, wobei Verbindungen der Formel IX ausgenommen sind, worin n = 1 ist, $R^3$ Wasserstoff oder Ethyl bedeutet und $Z^1$, $Z^2$ jeweils für Wasserstoff steht, dadurch gekennzeichnet, daß man Verbindungen der Formel IV

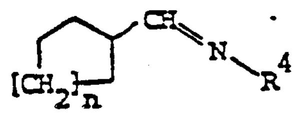

(IV)

in welcher n die vorstehende Bedeutung hat und $R^4$ für Alkyl mit 1 bis 6 C-Atomen steht, mit einem metallorganischen Reagenz in einem inerten Lösungsmittel metalliert und anschließend mit Verbindungen der Formel V

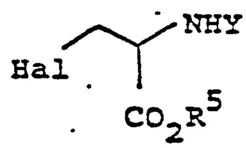

(V)

in welcher Hal Brom oder Chlor, $R^5$ Alkyl mit 1 bis 6 C-Atomen oder Aralkyl mit 7 bis 9 C-Atomen und Y einen sauer abspaltbaren aliphatischen oder aromatischen Acylrest bedeuten, oder mit Verbindungen der Formel VI,

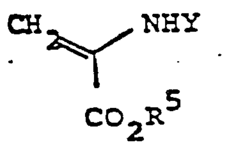

(VI)

in welcher $R^5$ und Y vorstehende Bedeutung haben, zu Verbindungen der Formel VII,

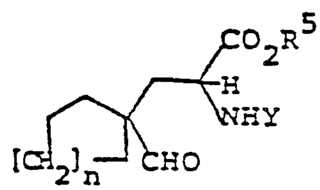

(VII)

in welcher n, $R^5$ und Y vorstehende Bedeutung haben, umsetzt, diese durch Säurebehandlung zu Verbindungen der Formel IX, in welcher n die vorstehende Bedeutung hat, $R^3$ für Wasserstoff und $Z^1 + Z^2$ gemeinsam für eine chemische Bindung stehen, cyclisiert, diese zu Verbindungen der Formel IX, in welcher n vorstehende Bedeutung hat und $R^3$ für Wasserstoff steht und $Z^1$, $Z^2$ jeweils Wasserstoff bedeutet, reduziert und diese anschließend gegebenenfalls zu Verbindungen der Formel IX, in welcher n, $Z^1$ und $Z^2$ vorstehende Bedeutung und $R^3$ die im Anspruch 1 angegebene Bedeutung mit Ausnahme der von Wasserstoff haben, verestert.

5. Verfahren gemäß einem der Ansprüche 1 bis 3 zur Herstellung einer Verbindung der Formel I zur Verwendung als Heilmittel.

6. Verfahren zur Herstellung eines Anneimittels, enthaltend eine Verbindung der Formel I, dadurch gekennzeichnet, daß man die Verbindung der Formel I in eine geeignete Zubereitungsform bringt.

7. Verfahren zur Herstellung eines Anneimittels, enthaltend eine Verbindung der Formel I und ein Diuretikum, dadurch gekennzeichnet, daß man die Verbindung der Formel I zusammen mit einem Diuretikum in eine geeignete Zubereitungsform bringt.

**Claims** (for the Contracting States : BE, CH, DE, FR, GB, IT, LI, LU, NE, SE)

1. Compounds of the formula I

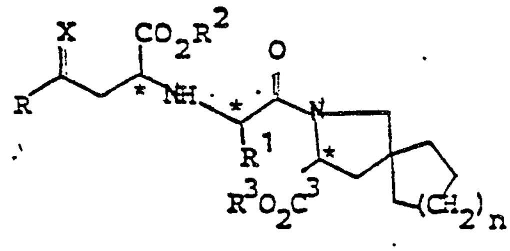

(I)

in which

n denotes 1, 2, 3 or 4,

R denotes phenyl,

$R^1$ denotes en alkyl radical of a naturally occurring amino acid HOOC—CH($NH_2$)—$R^1$,

$R^2$ denotes hydrogen, alkyl having 1 to 6 C atoms or optionally nitro-substituted aralkyl having 7 to 9 C atoms,

$R^3$ denotes hydrogen, alkyl having 1 to 10 C atoms, cycloalkyl having 3 to 10 C atoms or optionally nitro-substituted aralkyl having 7 to 9 C atoms and

X denotes 2 hydrogen atoms or 1 oxygen atom,

and its physiologically tolerated salts which acids and, if $R^2$ and/or $R^3$ denotes hydrogen, with bases, with the proviso that 2-[N-(1-carboethoxy-3-phenylpropyl)-(S)-alanyl]-2-azaspiro [4.4] nonane-3(S)-carboxylic acid and 2-[N-(1-carboethoxy-3-phenylpropyl)-(S)-alanyl]-2-azaspiro [4.5] decane-3(S)-carboxylic acid and physiologically tolerated salts thereof are excluded.

2. Compounds of the formula I as claimed in claim 1, characterized in that the C atom in the 3-position of the spirocycle and the C atoms marked with an asterisk in the chain each have the S configuration.

3. Compounds of the formula I as claimed in either of claims 1 or 2, characterized in that

$R^1$ denotes methyl,

$R^2$ denotes hydrogen, alkyl having 1 to 4 C atoms, benzyl or nitrobenzyl and

$R^3$ denotes hydrogen.

4. A process for the preparation of compounds of the formula I, which comprises condensing a compound of the formula II

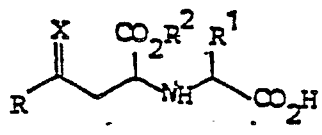

(II)

in which R, $R^1$, $R^2$ and X have the meanings indicated in claim 1, with a compound of the formula III

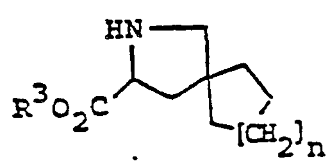

(III)

in which $R^3$ has the meaning indicated in claim 1, with the exception of hydrogen, and n represents a whole number from 1 to 4, and then optionally splitting off the radicals $R^2$ and/or $R^3$ by hydrogenolysis, or with acid or base, and optionally converting the compound of the formula I obtained into its physiologically tolerated salts.

5. A process for the preparation of compounds of the formula III in which n and $R^3$ have the meaning mentioned in claim 1, which comprises metalizing a compound of the formula IV

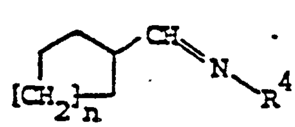

(IV)

in which n has the abovementioned meaning and $R^4$ represents alkyl having 1 to 6 C atoms, with an organometallic reagent in an inert solvent and then reacting with a compound of the formula V

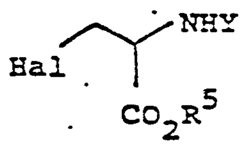

(V)

in which Hal denotes bromine or chlorine, $R^5$ denotes alkyl having 1 to 6 C atoms or aralkyl having 7 to 9 C atoms and Y denotes an aliphatic or aromatic acyl radical which can be split off by acid, or reacting with a compound of the formula VI

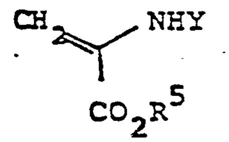

(VI)

in which $R^5$ and Y have the abovementioned meaning, to give a compound of the formula VII

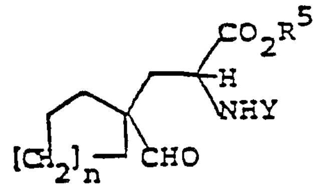

(VII)

in which n, $R^5$ and Y have the abovementioned meaning, cyclizing the latter by acid treatment to give a compound of the formula VIII

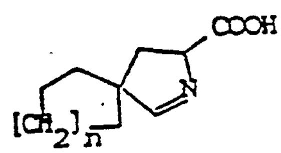

(VIII)

in which n has the abovementioned meaning, reducing the latter to give a compound of the formula III in which n has the abovementioned meaning and $R^3$ represents hydrogen, and then esterifying the latter to give a compound of the formula III in which n has the abovementioned meaning and $R^3$ has the meaning indicated in claim 1 with the exception of hydrogen.

6. A compound of the formula IX

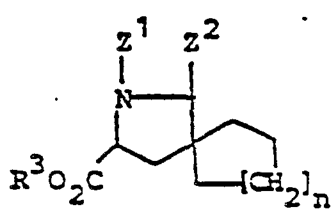

(IX)

in which n and $R^3$ have the meaning mentioned in claim 1 and $Z^1$, $Z^2$ each represent hydrogen or, together, represent a chemical bond, with the proviso that compounds of the formula IX are excluded, in which n is 1, $R^3$ denotes hydrogen or ethyl and $Z^1$, $Z^2$ each represent hydrogen.

7. Compounds of the formula IX as claimed in claim 6, wherein $Z^1$ and $Z^2$ denote hydrogen.

8. A compound as claimed in any of claims 1 to 3 for use as a medicament.

9. A compound as claimed in claim 8 for use in combination with a diuretic.

10. A pharmaceutical composition containing a compound as claimed in any of claims 1 to 3.

11. A pharmaceutical composition containing a compound as claimed in any of claims 1 to 3 and a diuretic.

**Claims** (for the Contracting State AT)

1. A process for the preparation of compounds of the formula I

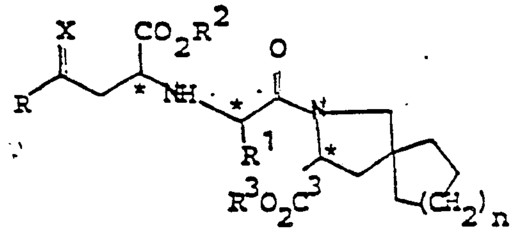

(I)

in which
n denotes 1, 2, 3 or 4,
R denotes phenyl,
$R^1$ denotes an alkyl radical of a naturally occurring amino acid HOOC—CH($NH_2$)—$R^1$,
$R^2$ denotes hydrogen, alkyl having 1 to 6 C atoms or optionally nitro-substituted aralkyl having 7 to 9 C atoms,
$R^3$ denotes hydrogen, alkyl having 1 to 10 C atoms, cycloalkyl having 3 to 10 C atoms or optionally nitro-substituted aralkyl having 7 to 9 C atoms and
X denotes 2 hydrogen atoms or 1 oxygen atom,
and its physiologically tolerated salts with acids and, if $R^2$ and/or $R^3$ denotes hydrogen, with bases, with the proviso that 2-[N-(1-carboethoxy-3-phenylpropyl)-(S)-alanyl]-2-azaspiro[4.4]nonane-3(S)-carboxylic acid and 2-[N-(1-carboethoxy-3-phenylpropyl)-(S)-alanyl]-2-azaspiro[4.5]decane-3(S)-carboxylic acid and physiologically tolerated salts thereof are excluded, which process comprises condensing a compound of the formula II

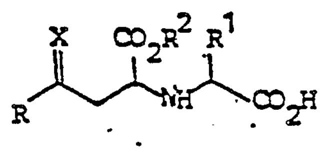

(II)

in which R, $R^1$, $R^2$ and X have the meanings indicated above, with a compound of the formula III

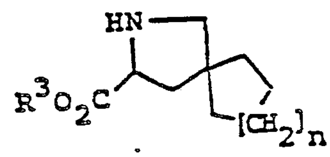

(III)

in which $R^3$ has the meaning indicated above, with the exception of hydrogen, and n represents a whole number from 1 to 4, and then optionally spliting off the radicals $R^2$ and/or $R^3$ by hydrogenolysis, or with acid or base, and optionally converting the compound of the formula I obtained into its physiologically tolerated salts.

2. A process as claimed in claim 1, characterized in that compounds of the formula I are prepared, wherein the C atom in the 3-position of the spirocycle and the C atoms marked with an asterisk in the chain each have the S configuration.

3. A process as claimed in either of claims 1 or 2, characterized in that in formula I
$R^1$ denotes methyl,
$R^2$ denotes hydrogen, alkyl having 1 to 4 C atoms, benzyl or nitrobenzyl and
$R^3$ denotes hydrogen.

4. A process for the preparation of a compound of the formula III in which n and $R^3$ have the meaning mentioned in claim 1, which comprises metalizing a compound of the formula IV

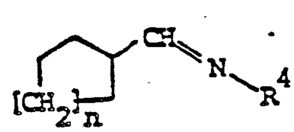

(IV)

in which n has the abovementioned meaning and $R^4$ represents alkyl having 1 to 6 C atoms, with an organometallic reagent in an inert solvent and then reacting with a compound of the formula V

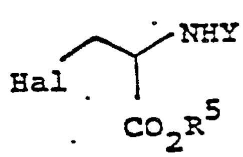

(V)

in which Hal denotes bromine or chlorine, $R^5$ denotes alkyl having 1 to 6 C atoms or aralkyl having 7 to 9 C atoms and Y denotes an aliphatic or aromatic acyl radical which can be splitt off by acid, or reacting with a compound of the formula VI

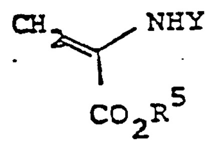

(VI)

in which $R^5$ and Y have the abovementioned meaning, to give a compound of the formula VII

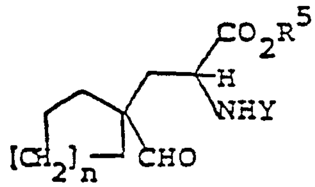

(VII)

in which n, $R^5$ and Y have the abovementioned meaning, cyclizing the latter by acid treatment to give a compound of the formula VIII

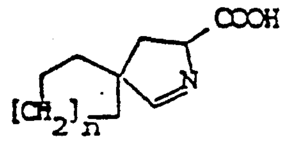

(VIII)

in which n has the abovementioned meaning, reducing the latter to give a compound of the formula III in which n has the abovementioned meaning and $R^3$ represents hydrogen, and then esterifying the latter to give a compound of the formula III in which n has the abovementioned meaning and $R^3$ has the meaning indicated in claim 1 with the exception of hydrogen.

5. A process as claimed in any of claims 1 to 3 for preparing a compound of the formula I for use as a medicament.

6. A process for preparing a pharmaceutical composition containing a compound of the formula I, characterized in that the compound of the formula I is brought in a suitable form for administration.

7. A process for preparing a pharmaceutical composition containing a compound of the formula I and a diuretic, characterized in that the compound of the formula I together with the diuretic is brought in a suitable form for administration.

**Revendications** (pour les Etats contractants : BE, CH, DE, FR, GB, IT, LI, LU, NL, SE)

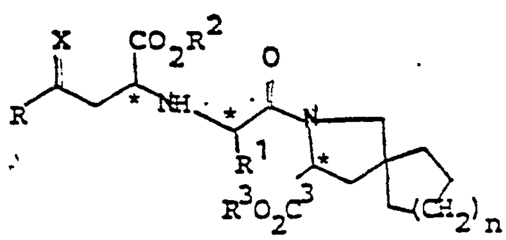

(I)

dans laquelle :

n représente 1, 2, 3 ou 4,

R représente un groupe phényle,

$R^1$ représente un groupe alcoyle d'un aminoacide naturel HOOC—CH($NH_2$)—$R^1$,

$R^2$ représente l'hydrogène, un groupe alcoyle en $C_1$ à $C_6$ ou aralcoyle en $C_7$ à $C_9$, le cas échéant nitrosubstitué,

$R^3$ représente l'hydrogène, un groupe alcoyle en $C_1$ à $C_{10}$, un groupe cycloalcoyle en $C_3$ à $C_{10}$ ou un groupe aralcoyle en $C_7$ à $C_9$, le cas échéant nitrosubstitué,

X représente deux atomes d'hydrogène ou un atome d'oxygène,

ainsi que leurs sels physiologiquement acceptables avec des acides et, si $R^2$ et/ou $R^3$ désignent l'hydrogène, avec des bases, des acides 2-[N-(1-éthoxycarbonyle-3-phényl-propyle)-(S)-alanyle]-2-azaspiro-[4.4]-nonane-3(S)-carboxylique et 2-[N-(1-éthoxycarbonyle-3-phénylpropyle)-(S)-alanyle]-2-azaspiro-[4.5]-décane-3(S)-carboxylique ainsi que leurs sels physiologiquement acceptables étant exclus.

2. Composés répondant à la formule I selon la revendication 1, caractérisés en ce que l'atome de carbone en position 3 du cycle spiro ainsi que les atomes de carbone de la chaîne marqués d'un astérique

présentent à chaque fois la configuration S.

3. Composés répondant à la formule I selon la revendication 1, caractérisés en ce que

$R^1$ représente un groupe méthyle,

$R^2$ représente l'hydrogène, un groupe alcoyle en $C_1$ à $C_4$, benzyle ou nitrobenzyle, et

$R^3$ représente l'hydrogène.

4. Procédé de préparation de composés répondant à la formule I, caractérisé en ce qu'on condense des composés répondant à la formule II :

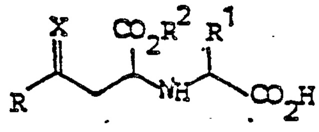

(II)

dans laquelle $R^1$, $R^2$ et X ont les significations indiquées dans la revendication 1, avec des composés répondant à la formule III :

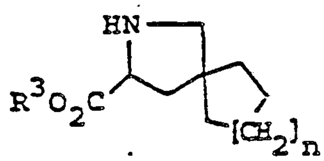

(III)

dans laquelle $R^3$ a la signification indiquée dans la revendication 1 à l'exception de l'hydrogène et n désigne un nombre entier de 1 à 4 ; puis on élimine le cas échéant les radicaux $R^2$ et/ou $R^3$ par hydrogénolyse, par action d'un acide ou d'une base et on transforme le cas échéant les composés répondant à la formule I obtenus en leurs sels physiologiquement acceptables.

5. Un procédé de préparation de composés répondant à la formule III, dans laquelle n et $R^3$ ont les significations indiquées dans la revendication 1, caractérisé en ce qu'on métallise des composés répondant à la formule IV :

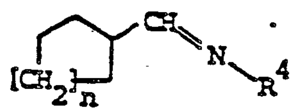

(IV)

dans laquelle n a la signification précédemment indiquée et $R^4$ désigne un groupe alcoyle en $C_1$ à $C_6$, avec un réactif organométallique dans un solvant inerte, puis on les fait réagir avec des composés répondant à la formule V :

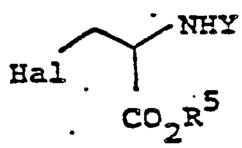

(V)

dans laquelle Hal désigne le brome ou le chlore, $R^5$ un groupe alcoyle en $C_1$ à $C_6$ ou aralcoyle en $C_7$ à $C_9$ et Y un radical acyle aliphatique ou aromatique éliminable par les acides, avec des composés répondant à la formule VI :

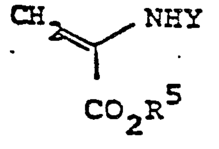

(VI)

dans laquelle $R_5$ et Y ont les significations précédemment indiquées, pour donner des composés répondant à la formule VII :

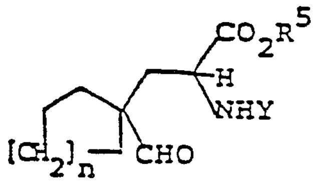

(VII)

dans laquelle n, $R^5$ et Y ont les significations précédentes, on les cyclise par traitement acide en composés répondant à la formule VIII :

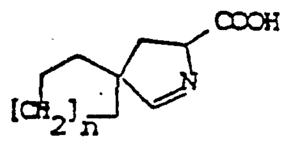

(VIII)

dans laquelle n a la signification précédente, on les réduit en composés répondant à la formule III, dans laquelle n a la signification précédente et $R^3$ désigne l'hydrogène, puis on les estérifie en composés répondant à la formule III dans laquelle n a la signification précédente et $R^3$ a la signification indiquée dans la revendication 1, à l'exception de l'hydrogène.

6. Composés répondant à la formule IX

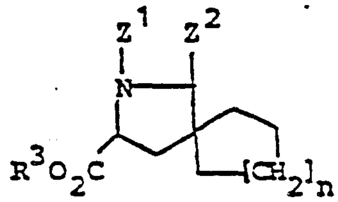

(IX)

dans laquelle n et $R^3$ ont la signification indiquée à la revendication 1 et $Z^1$ et $Z^2$ représentent chacun l'hydrogène ou représentent ensemble une liaison chimique, des composés répondant à la formule IX dans laquelle n représente 1, $R^3$ représente l'hydrogène ou un groupe éthyle et $Z^1$ et $Z^2$ représentent chacun l'hydrogène étant exclus.

7. Composés répondant à la formule IX selon la revendication 6, caractérisés en ce que $Z^1$ et $Z^2$ représentent l'hydrogène.

8. Composé selon l'une quelconque des revendications 1 à 3 à utiliser comme médicament.

9. Composé selon la revendication 8 à utiliser en association avec un diurétique.

10. Médicament contenant un composé suivant l'une quelconque des revendications 1 à 3.

11. Médicament contenant un composé suivant l'une quelconque des revendications 1 à 3 ainsi qu'un diurétique.

**Revendications** (pour l'Etat contractant AT)

1. Un procédé pour préparer des composés répondant à la formule I.

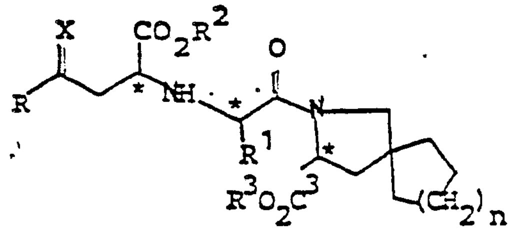

(I)

dans laquelle :

n représente 1, 2, 3 ou 4,

R représente un groupe phényle,

$R^1$ représente un groupe alcoyle d'un aminoacide naturel HOOC—CH($NH_2$)—$R^1$,

$R^2$ représente l'hydrogène, un groupe alcoyle en $C_1$ à $C_6$ ou aralcoyle en $C_7$ à $C_9$, le cas échéant nitrosubstitué,

$R^3$ représente l'hydrogène, un groupe alcoyle en $C_1$ à $C_{10}$, un groupe cycloalcoyle en $C_3$ à $C_{10}$ ou un groupe aralcoyle en $C_7$ à $C_9$, le cas échéant nitrosubstitué,

X représente deux atomes d'hydrogène ou un atome d'oxygène,

ainsi que leurs sels physiologiquement acceptables avec des acides et, si $R^2$ et/ou $R^3$ désignent l'hydrogène, avec des bases, des acides 2-[N-(1-éthoxycarbonyle-3-phényl-propyle)-(S)-alanyle]-2-azaspiro-[4.4]-nonane-3(S)-carboxylique et 2-[N-(1-éthoxycarbonyle-3-phénylpropyle)-(S)-alanyle]-2-azaspiro-[4.5]-décane-3(S)-carboxylique ainsi que leurs sels physiologiquement acceptables étant exclus, caractérisé en ce qu'on condense des composés répondant à la formule II :

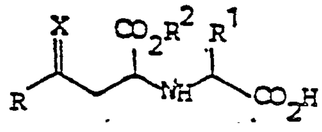

(II)

dans laquelle R, $R^1$, $R^2$ et X ont les significations indiquées dans la revendication 1, avec des composés répondant à la formule III :

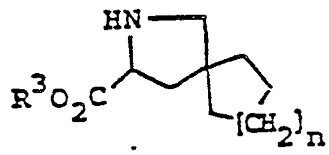

(III)

dans laquelle $R^3$ a la signification indiquée ci-dessus à l'exception de l'hydrogène et n désigne un nombre entier de 1 à 4 ; puis on élimine le cas échéant les radicaux $R^2$ et/ou $R^3$ par hydrogénolyse, par action d'un acide ou d'une base et on transforme le cas échéant les composés répondant à la formule I obtenus en leurs sels physiologiquement acceptables.

2. Un procédé selon la revendication 1, caractérisé en ce qu'on prépare des composés répondant à la formule I, dans lesquels l'atome de carbone en position 3 du cycle spiro ainsi que les atomes de carbone de la chaîne marqués d'un astérisque présentent à chaque fois la configuration S.

3. Un procédé selon la revendication 1, caractérisé en ce que dans la formule I
$R^1$ représente un groupe méthyle,
$R^2$ représente l'hydrogène, un groupe alcoyle en $C_1$ à $C_4$, benzyle ou nitrobenzyle, et
$R^3$ représente l'hydrogène

4. Procédé de préparation de composés répondant à la formule IX

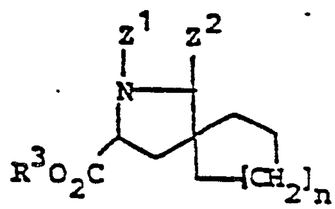

(IX)

dans laquelle n et $R^3$ ont la signification indiquée à la revendication 1 et $Z^1$ et $Z^2$ représentent chacun l'hydrogène ou représentent ensemble une liaison chimique, des composés répondant à la formule IX dans laquelle n représente 1, $R^3$ représente l'hydrogène ou un groupe éthyle et $Z^1$ et $Z^2$ représentent chacun l'hydrogène étant exclus,
caractérisé en ce qu'on métallise des composés répondant à la formule IV :

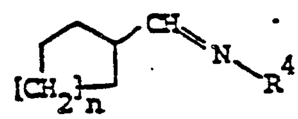

(IV)

dans laquelle n a la signification précédemment indiquée et $R^4$ désigne un groupe alcoyle en $C_1$ à $C_6$, avec un réactif organométallique dans un solvant inerte, puis on les fait réagir avec des composés répondant à la formule V :

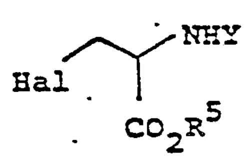

(V)

dans laquelle Hal désigne le brome ou le chlore, $R^5$ un groupe alcoyle en $C_1$ à $C_6$ ou aralcoyle en $C_7$ à $C_9$ et Y un radical acyle aliphatique ou aromatique éliminable par les acides,
avec des composés répondant à la formule VI :

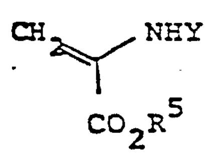

(VI)

dans laquelle $R^5$ et Y ont les significations précédemment indiquées, pour donner des composés répondant à la formule VII :

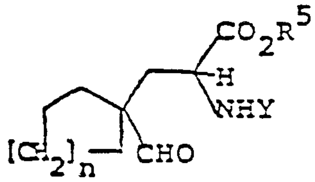

(VII)

dans laquelle n, $R^5$ et Y ont les significations précédentes, on les cyclise par traitement acide en composés répondant à la formule IX :

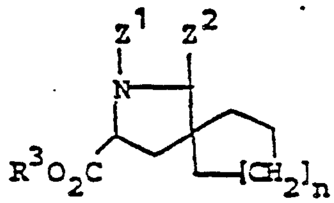

(IX)

dans laquelle n a la signification précédente, $R^3$ désigne l'hydrogène et $Z^1$ et $Z^2$ représentent ensemble une liaison chimique, on les réduit en composés répondant à la formule IX, dans laquelle n a la signification précédente, $Z^1$ et $Z^2$ représentent chacun l'hydrogène et $R^3$ désigne l'hydrogène, puis on les estérifie le cas échéant en composés répondant à la formule IX dans laquelle $Z^1$ et $Z^2$ et n ont la signification précédente et $R^3$ a la signification indiquée dans la revendication 1, à l'exception de l'hydrogène.

5. Procédé suivant l'une quelconque des revendications 1 à 3 pour préparer un composé répondant à la formule I à utiliser comme médicament.

6. Procédé de préparation d'un médicament contenant un composé répondant à la formule I, caractérisé en ce qu'on met un composé répondant à la formule I sous une forme d'administration appropriée.

7. Procédé de préparation d'un médicament contenant un composé répondant à la formule I et un diurétique, caractérisé en ce qu'on met le composé répondant à la formule I sans une forme d'administration appropriée, en association avec un diurétique.